# EUROPÄISCHE PATENTSCHRIFT

(11) **EP 0 613 732 B1**
(45) Veröffentlichungstag und Bekanntmachung des Hinweises auf die Patenterteilung: **23.12.1998**
(21) Anmeldenummer: 94100214.9
(22) Anmeldetag: 08.01.1994
(51) Int. Cl.: B07C 5/34, B07C 5/12, G01N 21/90

(54) **Verfahren und Vorrichtung zum Überprüfen der Funktionsfähigkeit von Inspektionseinrichtungen an Flascheninspektionsmaschinen**
Method and device for testing the working capacity of inspection units in bottle inspecting machines
Procédé et dispositif pour tester la capacité de fonctionnement d'unités d'inspection dans des machines d'inspection de bouteilles

(30) Priorität: 30.01.1993 DE 4302656
(43) Veröffentlichungstag der Anmeldung: 07.09.1994
(73) Patentinhaber: KHS Maschinen- und Anlagenbau Aktiengesellschaft, 44143 Dortmund (DE)
(72) Erfinder: Pschicholz, Manfred, D-44310 Dortmund (DE); Gaisbauer, Hubert, D-58710 Menden (DE)

(56) Entgegenhaltungen:
- DE-A- 2 717 955
- FR-A- 2 551 046
- GB-A- 2 094 530

## Beschreibung

Die Erfindung bezieht sich auf ein Verfahren zum Überprüfen der Funktionsfähigkeit von Inspektionseinrichtungen an Flascheninspektionsmaschinen gemäß Oberbegriff des Anspruchs 1 sowie auf eine Vorrichtung zur Durchführung des Verfahrens. Ein derartiges Verfahren ist aus der DE-A-33 24 449 bekannt.

Flascheninspektionsmaschinen sind vorzugsweise als Rundläufer ausgebildet und mit verschiedenen Prüfvorrichtungen und Inspektionseinrichtungen ausgestattet, die von den Flaschen durchlaufen werden. Bei bekannten Flascheninspektionsmaschinen befinden sich oberhalb oder seitlich der Bewegungsebene der Flaschen mehrere Inspektionseinrichtungen, mit denen die verschiedenartigsten Schäden oder Verschmutzungen überprüft werden. Diese Inspektionseinrichtungen können im Laufe der Zeit unterschiedliche Reaktionscharakteristiken aufweisen, so daß es erforderlich ist, in einem bestimmten Zeitabschnitt alle Funktionen zu überprüfen und gegebenenfalls eine entsprechende Nachjustierung der einzelnen Prüfvorrichtungen vorzunehmen. Diese Methode ist sehr arbeitsaufwendig und kostenintensiv. Üblicherweise werden hierbei Testflaschen eingesetzt und von Hand in die normale Produktionsbahn eingegeben und durch die Inspektionsmaschine geführt.

Aus der DE-A-21 66 235 ist bereits eine derartige Vorrichtung zur selbsttätigen Überprüfung von Inspektionsmaschinen von Behältern, insbesondere von Flascheninspektionsmaschinen bekannt geworden, die einen oder eine Vielzahl am Umfang rotierender Fördermittel angeordnete Prüfköpfe sowie eine Zu- und eine Abtransportstrecke und eine Aussortierstation aufweisen, wobei die zu überprüfenden Flaschen in Reihen zugeführt und nach Durchlauf einer bestimmten Umfangstrecke zu einer Weiterbehandlungsmaschine abtransportiert werden. Die Inspektionsmaschine weist mindestens einen, eine künstliche Fehlerquelle darstellenden Prüfkörper auf, der in den Bereich der Prüfköpfe bringbar und anhand dessen die Funktion der Prüfköpfe überprüfbar ist.

Eine weitere Vorrichtung zur Überprüfung von Flaschen ist aus der DE-A-24 27 054 bekannt geworden, wobei jede Prüfstation mit der Feststellung eines Mangels über eine elektronische Einrichtung nach einer entsprechenden Verzögerungszeit ein Ausstoßsignal an eine hinter der Auswertestation gelegene Ausstoßvorrichtung abgibt. Eine Eigenüberprüfung dieser Vorrichtung mit Prüfflaschen ist nicht vorgesehen.

Ferner ist aus der eingangs genannten DE-A-33 24 449 eine Vorrichtung zur Überprüfung von Inspektionsmaschinen für Behälter, insbesondere Flascheninspektionsmaschinen, bekannt geworden. Hierbei ist der Prüfkörper eine fehlerhafte Flasche, die der Inspektionsmaschine zugeleitet wird. Eine als nicht fehlerhaft erkannte Flasche signalisiert eine Fehlfunktion der entsprechenden Inspektionseinrichtung. Als Prüfflaschen werden codierbare oder mit einem Erkennungs- und/oder lesbaren Teststreifen ausgestattete Behälter verwendet, deren Art automatisch ermittelbar ist. Hierbei ist es erforderlich, daß die Inspektionsmaschine auf Überprüfen geschaltet wird. Die Testflaschen werden dann automatisch zugeleitet. Es ist allerdings nicht erkennbar, welche Testflasche für welche Inspektionseinrichtung gewählt worden ist, so daß beispielsweise bei Fehlen ein oder mehrerer Testflaschen das betreffende System nicht überprüft wird und dieser Mangel nicht erkennbar gemacht werden kann. Es wird also lediglich ein Summensignal vermittelt.

Aufgabe der vorliegenden Erfindung ist es, hier eine Verbesserung zu schaffen, mit welcher gezielt die zu überprüfenden Inspektionseinrichtungen in Verbindung mit den zugeordneten Testflaschen aktiviert, ausgewertet und überwacht werden können.

Diese Aufgabe wird bei einem Verfahren der eingangs genannten Art dadurch gelöst, daß Erkennungssystem und Inspektionseinrichtungen einander zugeordnet sind, Erkennungssignal einem Zuordnungs- und Überwachungssystem vermittelt wird und aufgrund des abgegebenen Erkennungssignals das Inspektionsergebnis der dem vom Erkennungssystem erkannten Testflaschentyp zugeordneten Inspektionseinrichtung überwacht und bei Nichtansprechen der betreffenden Inspektionseinrichtung ein Fehlersignal ausgelöst wird.

Dabei hat es sich als zweckmäßig erwiesen, wenn den Inspektionseinrichtungen jeweils ein Erkennungssystem zugeordnet ist.

Mit diesem vorgeschlagenen Verfahren ist es möglich, eine Testflasche zunächst zu erkennen, dann deren Zuordnung zu der betreffenden Inspektionseinrichtung oder weiteren vorzunehmen und speziell diese Inspektionseinrichtung oder -einrichtungen zu überwachen. Dabei wird beispielsweise eine allgemeine Information übermittelt, welche dokumentiert, welche jeweiligen Inspektionseinrichtungen durch den Überprüfungsvorgang angesprochen worden sind, so daß genaue Daten vorhanden sind, ob und in welchem Umfang eine Eigenüberprüfung der Inspektionseinrichtungen vorgenommen worden ist.

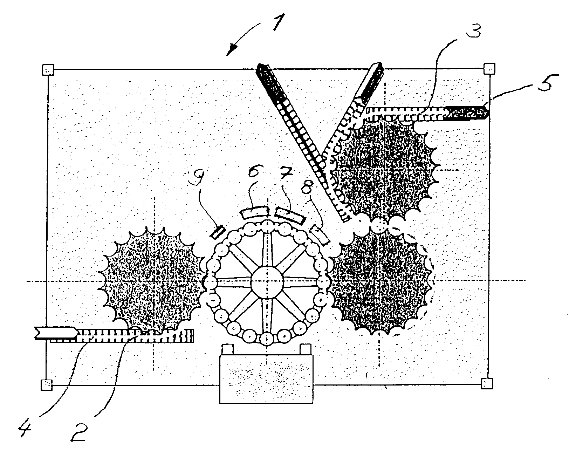

Im nachfolgenden wird die Erfindung anhand eines in der Zeichnung dargestellten Ausführungsbeispiels einer Vorrichtung zur Durchführung des angegebenen Verfahrens näher erläutert.

Gemäß dem Ausführungsbeispiel besteht die Flascheninspektionsmaschine 1 aus einem einlaufseitigen Bereich 2 und einem auslaufseitigen Bereich 3. Im einlaufseitigen Bereich 2 ist eine Zutransportstrecke 4 und im auslaufseitigen Bereich 3 eine Abtransportstrecke 5 für die zu überprüfenden Flaschen vorgesehen. Die jeweiligen Testflaschen werden im einlaufseitigen Bereich 2 zugeführt und durch die Inspektionsmaschine 1 geleitet und anschließend abtransportiert. Gemäß dem dargestellten Ausführungsbeispiel ist im Bereich oder vor den betreffenden Inspektionseinrichtungen 6, 7, 8 ein zweckmäßig für alle Testflaschen geeignetes Erkennungssystem 9 angeordnet, welches beim Vorbeitransport einer Testflasche ein entsprechendes Erkennungssignal abgibt und damit die entsprechende Inspektionseinrichtung 6, 7, 8 auf Überwachung aktiviert. Es wird also unmittelbar auf die zugeordnete Inspektionseinrichtung 6, 7, 8 eingewirkt und deren Inspektionsergebnis überwacht. Die erkannte Testflasche durchläuft die Flascheninspektionsmaschine 1 weiter und gelangt schließlich zu der betreffenden Inspektionseinrichtung 6, 7, 8, die bei einwandfreier Funktion ein Aussortiersignal für die betreffende Testflasche auslöst. Soweit dies nicht der Fall ist, wird von einem Überwachungssystem ein entsprechendes Fehlersignal ausgelöst, welches erkennen läßt, daß die gerade überprüfte Inspektionseinrichtung 6, 7, 8 nicht auf die für sie zuständige Testflasche angesprochen hat. Als Erkennungssystem kann ein im Zulaufbereich der Testflaschen auf deren Markierung ansprechender Sensor angeordnet sein, dessen Signal dem Zuordnungs -und Überwachungssystem zugeleitet wird.

Der Sensor kann als Lichtempfänger mit Lichtleiter ausgebildet sein.

Es ist aber auch denkbar, den Inspektionseinrichtungen 6, 7, 8 selbst jeweils ein Erkennungssystem speziell zuzuordnen, durch welches die Inspektionseinrichtung bei Durchlauf einer zugeordneten Testflasche eigenständig überwacht und bei Nichtansprechen ein entsprechendes Fehlersignal für diese bestimmte Inspektionseinrichtung 6, 7, 8 ausgelöst wird.

Allerdings ist es auch möglich, zunächst eine Inspektion der Testflasche vorzunehmen, dann deren Markierung zu ermitteln und mit dem Ergebnis der jeweiligen Inspektionseinrichtung zu vergleichen. Auf diese Weise kann ebenfalls festgestellt werden, ob das oder die durch die Testflasche vorgegebenen Fehler oder Beschädigungen von der zuständigen Inspektionseinrichtung erkannt worden sind.

## Patentansprüche

1. Verfahren zum Überprüfen der Funktionsfähigkeit von Inspektionseinrichtungen (6,7,8) an Flascheninspektionsmaschinen, bei welchem Testflaschen unterschiedlichen Typs mit entsprechenden Markierungen durch die Flascheninspektionsmaschine geleitet und von den zugeordneten Inspektionseinrichtungen (6,7,8) überprüft werden, wobei von einem Erkennungssystem (9) ein dem jeweiligen Testflaschentyp entsprechendes Erkennungssignal abgegeben wird, ***dadurch gekennzeichnet***, daß Erkennungssystem (9) und Inspektionseinrichtungen (6,7,8) einander zugeordnet sind, das Erkennungssignal einem Zuordnungs- und Überwachungssystem vermittelt wird und aufgrund des abgegebenen Erkennungssignals das Inspektionsergebnis der dem vom Erkennungssystem (9) erkannten Testflaschentyp zugeordneten Inspektionseinrichtung überwacht und bei Nichtansprechen der betreffenden Insektionseinrichtung ein Fehlersignal ausgelöst wird.

2. Verfahren nach Anspruch 1, ***dadurch gekennzeichnet***, daß den Inspektionseinrichtungen (6,7,8) jeweils ein Erkennungssystem (9) zugeordnet ist.

3. Vorrichtung zum Überprüfen der Funktionsfähigkeit von Inspektionsenrchtungen an Flascheninspektionsmaschinen, ***dadurch gekennzeichnet***, daß im Transportwegbereich von Testflaschen ein deren unterschiedliche Markierung erkennender Sensor und ein Zuordnungs- und Überwachungssystem angeordnet ist, dem die Sensorsignale zugeleitet werden und welches das Inspektionsergebnis der dem erkannten Testflaschentyp zugeordneten Inspektionseinrichtung überwacht.

4. Vorrichtung zum Überprüfen der Funktionsfähigkeit von Inspektionseinrichtungen an Flascheninspektionsmaschinen, ***dadurch gekennzeichnet***, daß im Transportwegbereich von Testflaschen deren unterschiedliche Markierung erkennende Sensoren angeordnet sind, die den Inspektionseinrichtungen jeweils speziell zugeordnet sind und mittels derer die jeweilige Inspektionseinrichtung bei Durchlauf einer zugeordneten Testflasche eigenständig überwacht wird.

5. Vorrichtung nach Anspruch 3 oder 4, ***dadurch gekennzeichnet***, daß als Sensor ein Lichtempfänger mit Lichtleiter dient.

## Claims

1. Method for testing the working capacity of inspection units (6,7,8) in bottle inspecting machines wherein test bottles of various types with respective markings pass through the bottle inspection machine and are tested by the associated inspection units (6,7,8), wherein an identification system (9) emits an identification signal corresponding to the respective test bottle type, characterised by the fact that the identification system (9) and the inspection units (6,7,8) are associated with each other, and wherein the identification signal is transferred to a coordination and control system, wherein the inspection result of the inspection unit associated with the test bottle type recognised by the identification system (9) is controlled and in case of non-operation of the respective inspection unit, an error signal is triggered off.

2. Method as claimed in claim 1, characterised by the fact that the inspection units (6,7,8) are provided with one identification system (9) each.

3. Device for testing the working capacity of inspection units in bottle inspecting machines, characterised by the fact that the conveying route of the test bottles is provided with a sensor and a coordination and control system and identifying the respective markings to which (system) the sensor signals are transmitted to, and which controls the inspection result of the inspection unit associated with the identified test bottle type.

4. Method and device for testing the working capacity of inspection units in bottle inspecting machines, characterised by the fact that the conveying route of test bottles are provided with sensors identifying the respective markings, each (sensor) being specially associated with the inspection unit and with the help of which the respective inspection unit is independently controlled during passage of an associated test bottle.

5. Device as claimed in claim 3 or 4, characterised by the fact that a light receiver with a photo conductor serves as sensor.

## Revendications

1. Procédé de vérification de l'aptitude à fonctionner de dispositifs d'inspection (6, 7, 8) sur des machines d'inspection de bouteilles, dans lequel des bouteilles test de différents types, portant des marquages correspondants, sont guidées à travers la machine d'inspection de bouteilles et sont vérifiées par les dispositifs d'inspection (6, 7, 8) associés, un signal de reconnaissance, correspondant à chaque type de bouteille test, étant émis par un système de reconnaissance (9), caractérisé en ce que le système de reconnaissance (9) et les dispositifs d'inspection (6, 7, 8) sont associés l'un à l'autre, en ce que le signal de reconnaissance est transmis à un système d'attribution et de surveillance et, sur la base du signal de reconnaissance émis, le résultat d'inspection du dispositif d'inspection associé au type de bouteille test reconnu par le système de reconnaissance (9) est surveillé et en ce qu'en cas d'absence de réaction du dispositif d'inspection correspondant, un signal de défaut est déclenché.

2. Procédé selon la revendication 1, caractérisé en ce qu'à chaque dispositif d'inspection (6, 7, 8) est associé un système de reconnaissance (9).

3. Dispositif de vérification de l'aptitude à fonctionner de dispositifs d'inspection sur des machines d'inspection de bouteilles, caractérisé en ce que, dans la zone de la trajectoire de transport des bouteilles test, est placé un capteur qui reconnaît leurs différents marquages et un système d'attribution et de surveillance, auquel sont envoyés les signaux du capteur, et qui surveille le résultat d'inspection du dispositif d'inspection associé au type de bouteille test qui a été reconnu.

4. Dispositif de vérification de l'aptitude à fonctionner de dispositifs d'inspection sur des machines d'inspection de bouteilles, caractérisé en ce que, dans la zone de la trajectoire de transport des bouteilles test, sont placés des capteurs qui reconnaissent leurs différents marquages, capteurs qui sont spécialement associés, chacun, aux dispositifs d'inspection et au moyen desquels le dispositif d'inspection correspondant est surveillé de façon autonome lorsque passe une bouteille test associée.

5. Dispositif selon la revendication 3 ou 4, caractérisé en ce qu'un élément photosensible comportant un guide de lumière sert de capteur.
